# EUROPEAN PATENT APPLICATION

(11) **EP 0 830 870 A1**
(43) Date of publication of application: **25.03.1998**
(21) Application number: 97202923.5
(22) Date of filing: 24.09.1997
(51) Int. Cl.: A61M 25/01, A61M 25/00

(54) **Catheter**

(30) Priority: 24.09.1996 NL 1004102
(71) Applicant: CORDIS EUROPA N.V., NL-9301 LJ Roden (NL)
(72) Inventor: Nap, Cornelis Philipus, 9345 AC Zevenhuizen (NL); Mous, Frans, 9402 HP Drachten (NL); Hurtak, Wenzel Franz, 9301 TZ Roden (NL)
(74) Representative: 't Jong, Bastiaan Jacobus

(57) **Abstract**

This invention relates to a catheter comprising a tube-like basic body with a distal end and a proximal end and at least one lumen extending through the basic body, which is connected with a connecting member at the proximal end, characterised by at least one cavity connected to the lumen at least close to the distal end.

## Description

This invention relates to a catheter comprising a tube-like basic body with a distal end and a proximal end and at least one lumen extending through the basic body, which is connected at the proximal end with a connecting member.

The disadvantage of the catheter according to prior techniques is that, along its length, both physical and mechanical properties cannot be affected. Varying such properties is often required however, for instance when visualization of the catheter by means of imaging using an MRI-technique or X-radiation is required.

The object of the invention is to remove at least the above-mentioned drawback, and to provide to this end a catheter which distinguishes itself by having at least one cavity connected with the lumen at least close to the distal end.

With a catheter according to this invention both the physical and mechanical properties of the catheter can be influenced. By filling for instance the cavity with a contrast medium, this catheter may be visualised by means of for instance imaging using X-radiation or an MRI-technique. In the case of a number of cavities positioned at a previously determined distance from one another, the cavities filled with a contrast medium serve as marking elements in the image to be visualised. By filling the cavity under pressure with a fluid such as air or liquid, the stiffness and the shape can for instance be adapted to requirements related to the application of the catheter. Also the shape of the catheter can be influenced by the shape and the size, as well as the orientation of the cavity which is filled under pressure with a fluid. A cavity located at for instance one side of the catheter and arranged in the flexible material of which the wall of the basic body has been made, causes bending of the catheter in relation to the basic body in the direction opposite to the cavity.

In a first embodiment, a catheter according to the invention is such that the cavity is ring-shaped. In this case the cavity has been arranged so as to surround the lumen.

A second embodiment comprises a catheter according to this invention with an additional lumen, which has been connected at the proximal end to a corresponding connecting member. The additional lumen is used in this case for other purposes, such as conveying a fluid through the catheter or receiving a device such as a glass fibre cable.

In a third embodiment a catheter according to this invention is such that a return lumen has been arranged which is connected with the cavity. When the cavity is filled with a fluid such as a contrast medium, air or another liquid which is to be removed from the cavity can be drained off again at the proximal end of the catheter and does consequently not have to be discharged inside the body into which the catheter has been introduced.

In a fourth embodiment a catheter according to this invention is such that the cavity is connected to an outlet at least close to the distal end. In this case the cavity may for instance serve as a temporary storage area for a fluid which is to be delivered inside the body into which the catheter has been introduced, at a carefully determined location.

In a fifth embodiment a catheter according to this invention is such that the cavity has been formed in between the tube-like basic body and a tubular member arranged in the lumen thereof. The advantage of a catheter made in this way is that it has a simple configuration which can consequently be manufactured cheaply.

In a sixth embodiment a catheter according to this invention is such that the connecting member corresponding with the lumen is connected, when in use, with pumping means for the supply and removal of a fluid. This is in particular an advantage when a fluid is to be delivered at a carefully pre-determined pressure.

The fluids to be arranged inside the cavity may for instance be a contrast medium, used with imaging techniques such as MRI or when using X-radiation, air or a resin curing under the influence of visible or UV-light.

The invention will be explained in greater detail below with reference to the attached drawings of an embodiment thereof.
- Figure 1: shows a perspective view of a catheter according to this invention.
- Figure 2: shows a cut-away perspective view of a section of the catheter illustrated in figure 1.

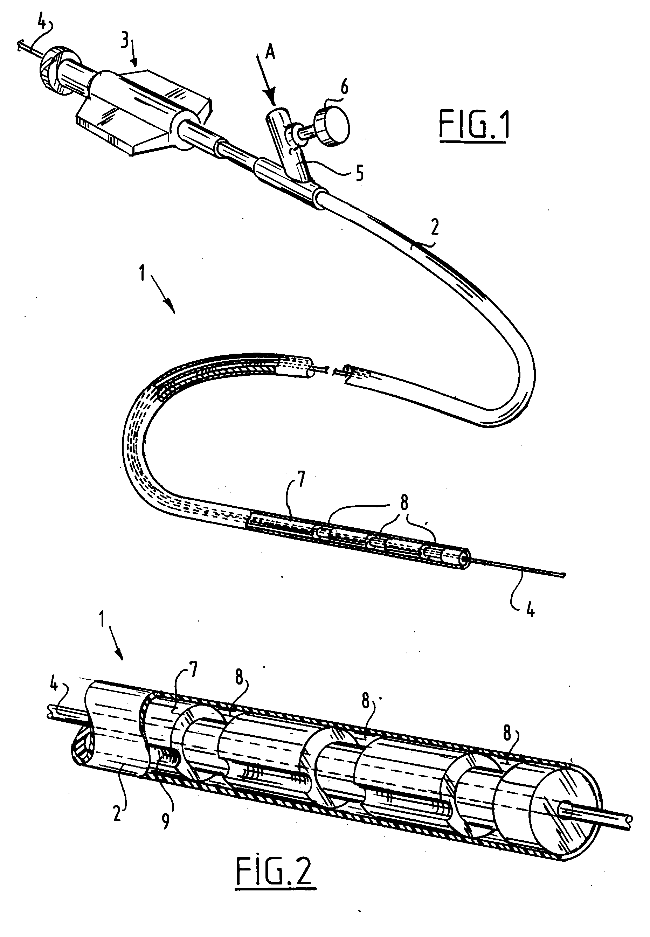

The catheter illustrated in figure 1 substantially comprises a tubular basic body in the shape of a hollow tube 2, a connecting member in the shape of a handle 3, and a tubular member in the shape of a filling body 7.

A guide wire 4 has been inserted through the handle 3, and has been advanced over the entire length of the catheter 1 through the hollow tube 2. The guide wire 4, being a glass fibre cable, also passes through the inside of the filling body 7 which has been illustrated in greater detail in figure 2.

It should be noted that the filling body 7 has a varying thickness, so that cavities 8 have been formed in between the filling body and the inside of the tube 2. The filling body also comprises an additional lumen shaped like a channel through which the glass fibre cable 4 has been inserted.

The cavities, which can be seen most clearly in figure 2, are connected to a connecting member in the shape of a Y-piece 5, which can be closed by means of the control means 6, in the open state of which fluid can be conveyed to the cavities 8 in the direction of the arrow A. A fluid to be transported to the cavity 8 may for instance be a contrast medium, whereby the distance between the separate cavities 8 is known in advance, so that when an image is created of the vicinity of the catheter 1 using X-radiation or an MRI-technique, the contrast medium designed for this purpose will be clearly visible, which will be helpful when interpreting the images because the distances between the cavities 8 are known beforehand. Furthermore, a fluid under pressure can be conveyed to the cavities 8 in order to increase the stiffness of the section of the catheter in which the cavities 8 have been arranged. In the case of another shape than the annular shape of the cavities 8 illustrated here, also bending of this section of the catheter 1 can be effected at any time during its use, which can also be neutralized again when it is no longer necessary, by reducing the pressure in the cavities 8. In addition, the same is true when contrast medium has been arranged inside the cavities 8, that is to say, it can be removed from the cavities again via the same route. Preferably a return lumen, not illustrated, has been arranged for this transport to and from the cavities 8, so that air or another liquid can take the place of the contrast medium or the fluid under pressure or vice versa, without having to open up a connection with the direct surroundings of the catheter.

In figure 2 a cut-away perspective view is shown of the tube 2 illustrated in figure 1, inside of which the filling body 7 has been arranged. The additional lumen and/or the return lumen have in this case been formed by parallel grooves 9 arranged, substantially in the longitudinal direction of the catheter, in the thicker sections of the filling body 7, the circumference of which thicker sections correspond as such to the cross-section of the tube 2, inside of which the filling body has been arranged.

By way of addition or as an alternative also other shapes of cavities than the annular cavities 8 described above and illustrated in the drawings can be employed. Spiral shaped cavities, winding cavities, circular cavities or drop shaped cavities may be used for example. This summing up is not exhaustive however; many other shapes may be employed. Particular attention should also be paid to asymmetric shapes, which serve to cause bending of the catheter 1 when the fluid is supplied to the cavities.

Although only air and liquid have been referred to above by way of fluids, it goes without saying that also other gasses than air may be used. Furthermore, separate cavities may have been provided with corresponding additional lumens and if desired also return lumens. As a result it will be possible to provide groups of cavities, which groups have been arranged dispersed around the circumference of the lumen, with for instance a gas under pressure in order to bend first in one direction, later in an opposite direction, etc.

Providing catheters with cavities offers in addition many other advantages, and they may have any number of shapes and may be arranged in a catheter in many different ways.

## Claims

1. Catheter comprising a tube-like basic body with a distal end and a proximal end and at least one lumen extending through the basic body, which is connected at the proximal end with a connecting member, characterised by at least one cavity connected with the lumen at least close to the distal end.

2. Catheter as claimed in claim 1, characterised in that the cavity is ring-shaped.

3. Catheter as claimed in claim 1 or 2, characterised by an additional lumen which is connected at the proximal end with a corresponding connecting member.

4. Catheter as claimed in claim 3, characterised in that the lumen has been arranged in a wall of the catheter next to the additional lumen.

5. Catheter as claimed in one of the previous claims, characterised in that a return lumen has been arranged which is connected to the cavity.

6. Catheter as claimed in one of the previous claims, characterised in that the cavity is connected with an outlet at least close to the distal end.

7. Catheter as claimed in one of the previous claims, characterised in that the cavity has been formed in between the tube-like basic body and a tubular body arranged inside it.

8. Catheter as claimed in claim 3, characterised in that the connecting member corresponding with the lumen is connected, when in use, with pumping means for the supply or removal of a fluid.

9. Catheter as claimed in claim 8, characterised in that the fluid comprises a contrast medium used for the purpose of imaging.

10. Catheter as claimed in claim 8, characterised in that the fluid comprises at least one gas, a gas mixture or a vapour.

11. Catheter as claimed in claim 8, characterised in that the fluid comprises a resin curing under the influence of visible or UV light.
